# FASCICULE DE BREVET EUROPEEN

(11) **EP 2 726 258 B1**
(45) Date de publication et mention de la délivrance du brevet: **30.03.2016**
(21) Numéro de dépôt: 12748754.4
(22) Date de dépôt: 29.06.2012
(51) Int. Cl.: B25J 9/04, B25J 18/00

(54) **SYSTEME ROBOTISE POUR LE DEPLACEMENT D'UN OUTIL GUIDE A DISTANCE**
ROBOTERSYSTEM ZUM VERSCHIEBEN EINES FERNGESTEUERTEN WERKZEUGS
ROBOTIZED SYSTEM FOR DISPLACING A REMOTELY GUIDED TOOL

(30) Priorité: 01.07.2011 FR 1102082
(43) Date de publication de la demande: 07.05.2014
(73) Titulaire: Advanced Echo Technology, 41310 Huisseau-en-Beauce (FR)
(72) Inventeur: LOUSTAUDAUDINE, Christophe, 41100 Areines (FR)
(74) Mandataire: Thibon-Littaye, Annick
(86) Numéro de dépôt international: PCT/IB2012/001290
(87) Numéro de publication internationale: WO 2013/005088

(56) Documents cités:
- EP-A2- 0 761 177
- FR-A1- 2 538 744
- FR-A1- 2 791 294
- US-A- 4 229 641

## Description

La présente invention concerne un système robotisé pour le déplacement d'un outil guidé à distance.

Le système robotisé selon l'invention s'applique notamment à la pratique d'examen échographique à distance, connu sous le terme de télé-échographie robotisé, dans lequel un examen d'échographie est réalisé sur un site isolé grâce à un robot présent sur ce site et téléopéré à distance par un médecin qualifié. Les mouvements de la main du médecin, qui commande un « joysticK » formant sonde fictive sont retransmis au niveau de la sonde réelle présente en bout du robot et appliquée sur le patient dans le site isolé. Le robot reproduit les mouvements imprimés par le médecin sensiblement en temps réel et le médecin reçoit sur un moniteur de contrôle l'image captée par la sonde réelle.

On connaît du document FR 2 791 294 un robot selon le préambule de la revendication 1 qui comporte un plateau avec un arbre, un porte satellite mobile en rotation autour de l'axe de l'arbre, un deuxième arbre monté à rotation sur le porte satellite, un porte outil monté sur le deuxième arbre, l'outil étant monté à rotation dans le porte outil.

Dans son mode de réalisation principal, ce robot est utilisé pour un mouvement à trois composantes de rotation d'une sonde échographique autour d'un point fixe qui est matérialisé par l'extrémité de la sonde au contact du corps du patient.

Il est prévu en outre un support pour le robot, avec le support qui permet d'effectuer des translations dans un plan horizontal passant par le point fixe. Ce déplacement en translation est assuré par quatre roues dentées disposées deux à deux symétriquement autour du support et dont l'excentricité du montage permet une translation dans une direction donnée et dans sa direction perpendiculaire pour transcrire le mouvement de translation de la sonde fictive en des coordonnées cartésiennes reproductibles.

Dans ce contexte, l'invention vise à améliorer un système robotisé pour la pratique notamment d'examen échographique, en proposant un système robotisé adapté à suivre les déplacements de rotation et de translation d'une sonde fictive dirigée par une personne qualifiée.

L'invention propose à cet effet un système robotisé selon la revendication 1 dans lequel un support sur lequel est rapporté un ensemble de bras articulés à l'extrémité duquel est disposé un outil adapté à être en contact avec une surface de travail déterminée, et dans lequel l'outil peut être déplacé en rotation autour de son point de contact avec la surface de travail, en conformité avec des instructions de déplacement en provenance d'un module de pilotage. Les instructions de déplacement sont calculées en fonction du déplacement à distance d'un outil de pilotage virtuel dudit système robotisé et elles sont ici remarquables en ce que d'une part le système est adapté à déplacer en translation le point de contact de l'outil avec la surface de travail et en ce que d'autre part cette translation de l'outil est commandée par le module de pilotage en cordonnées polaires, se matérialisant par un déplacement angulaire d'éléments constitutifs du support suivi d'un déplacement radial d'éléments constitutifs de ce même support

Ainsi, on peut proposer selon l'invention un système robotisé plus compact, de telle sorte que le champ d'application de ce système soit élargi. On pourra ainsi plus facilement appliquer ce système robotisé pour des appareillages de télé-échographie embarqués dans des navettes spatiales par exemple. Le système robotisé selon l'invention nécessite moins de débattement pour le déplacement d'un outil qu'il n'en était nécessaire jusqu'alors dans le cas d'un déplacement en translation selon des coordonnées cartésiennes.

On découple le mouvement de translation souhaité pour la sonde en des mouvements de rotation et de translation d'éléments constitutifs du support.

A cet effet, un mode de réalisation est tel que le support comporte une platine porteuse de l'extrémité de l'ensemble de bras articulés qui est montée en translation, pour la réalisation du déplacement radial, dans des rails solidaires d'un plateau adapté à tourner sur lui-même d'un angle déterminé, pour la réalisation du déplacement angulaire lors de la translation souhaitée de la sonde.

Selon différentes caractéristiques que l'on retrouve combinées dans un mode de réalisation présenté ci-après, la platine porte un actionneur qui entraîne en rotation un pignon adapté à coopérer avec une crémaillère formée sur un des rails solidaires du plateau. Cet actionneur est piloté par un signal de commande en provenance du module de commande pour la réalisation du déplacement radial.

Le plateau comporte en outre un trou disposé entre les rails et par lequel passe un premier arbre de rotation monté pivotant sur le support et solidaire de l'ensemble de bras articulés, le trou présentant un diamètre adapté pour permettre ledit déplacement radial.

Selon une caractéristique particulièrement avantageuse de l'invention, le module de commande utilise la rotation de l'ensemble des bras articulés porteurs de l'outil pour compenser la rotation induite de l'outil par son déplacement angulaire lors de sa translation polaire.

Ainsi, l'outil disposé sur site en bout du robot garde en cours de déplacement la même orientation que l'outil virtuel manipulé par le médecin. Si cet outil virtuel est mis en rotation, l'outil robotisé l'est également alors que si cet outil virtuel est uniquement mis en translation, sans rotation, on commande un angle de rotation de l'outil robotisé venant en compensation de la rotation angulaire rendue nécessaire pour la transcription en coordonnées polaires de la translation de l'outil.

Selon une caractéristique de l'invention, les éléments constitutifs du support comportent en outre une base fixe d'un portique à l'intérieur duquel tourne le plateau, ladite base fixe portant un arbre d'entraînement qui est monté à rotation sur ladite base, qui est solidaire en rotation du plateau et qui est associé à un actionneur, ledit actionneur étant piloté par un signal de commande en provenance du module de commande pour la réalisation dudit déplacement angulaire.

On utilise ainsi un axe de rotation de l'ensemble de bras articulés, déjà utilisé par ailleurs, pour compenser la rotation de l'outil induite par la translation en coordonnées polaires.

Selon une caractéristique de l'invention, l'ensemble de bras articulés comporte au moins un bras supérieur qui est solidaire en rotation d'un premier arbre du système monté pivotant sur le support, le module de commande générant une rotation dudit bras supérieur pour compenser ladite rotation induite de l'outil, caractérisé en ce que l'axe de rotation du bras supérieur est parallèle à l'axe du déplacement angulaire du support. L'ensemble de bras articulés comporte en outre un bras intermédiaire solidaire d'un deuxième arbre de rotation monté pivotant à l'extrémité libre dudit bras supérieur; ledit bras intermédiaire portant un troisième arbre de rotation duquel est solidaire en rotation un bras porte-outil sur lequel est monté l'outil, de telle sorte que trois axes de rotation, définis respectivement par le premier arbre, le deuxième arbre et le troisième arbre de rotation, sont concourants en l'extrémité de l'outil. Le module de commande est adapté à fournir d'une part des signaux de commande à destination d'actionneurs associés respectivement à chacun des bras articulés pour piloter la rotation des bras articulés autour de chacun des trois axes afin de commander le déplacement en rotation de l'outil et à fournir d'autre part un signal complémentaire inverse au signal correspondant au déplacement angulaire du bras supérieur pour compenser la rotation de l'outil induite par le déplacement angulaire dudit support pour le déplacement en translation de l'outil.

Selon une caractéristique de l'invention, chaque actionneur est associé à un capteur de position angulaire adapté à déterminer la position angulaire absolue du bras, de l'arbre ou du pignon piloté par ledit actionneur.

L'invention concerne également un dispositif de télé-échographie dans lequel on réalise à distance le déplacement d'une sonde d'échographie, le dispositif de télé-échographie comportant d'une part du matériel sur un site d'expertise où un expert médical peut diriger à distance l'échographie par l'intermédiaire d'une sonde virtuelle adaptée à transmettre des informations au module de commande du système robotisé afin de piloter le déplacement de l'outil, et d'autre part du matériel embarqué dans un site opératoire où se trouve le patient à examiner, le site opératoire comportant un système robotisé selon la présente invention adapté à réaliser l'échographie sur le patient en fonction des informations de commande qui découlent du déplacement de la sonde virtuelle sur le site d'expertise.

D'autres caractéristiques et avantages de l'invention ressortiront à la lecture de la description qui suit d'un de ses modes de réalisation, illustrée par:
- la figure 1 qui est une illustration schématique d'un principe de télé-échographie dans lequel s'inscrit le système robotisé selon l'invention dans un mode de réalisation particulier;
- la figure 2 qui est une vue en perspective d'un système robotisé selon l'invention dans le cadre de l'application à un système de télé-échographie illustrée sur la figure 1,
- la figure 3 qui est une vue en perspective, de dessus, du système illustré à la figure 2,
- la figure 4 qui est un agrandissement du dispositif de guidage en translation du système de la figure 2, avec la représentation plus détaillée du portique et de la platine, vu de dessous,
- la figure 5 qui est une vue partielle de l'agrandissement de la figure 3, sans le portique,
- et la figure 6 qui est un agrandissement d'un détail de la figure 2.

Dans ce qui suit, sans en limiter en quoi que ce soit la portée, on se placera ci-après dans le cadre de l'application préférée de l'invention, sauf mention contraire, c'est-à-dire dans le cas d'un robot de télé-échographie, piloté à distance par un professionnel de la santé.

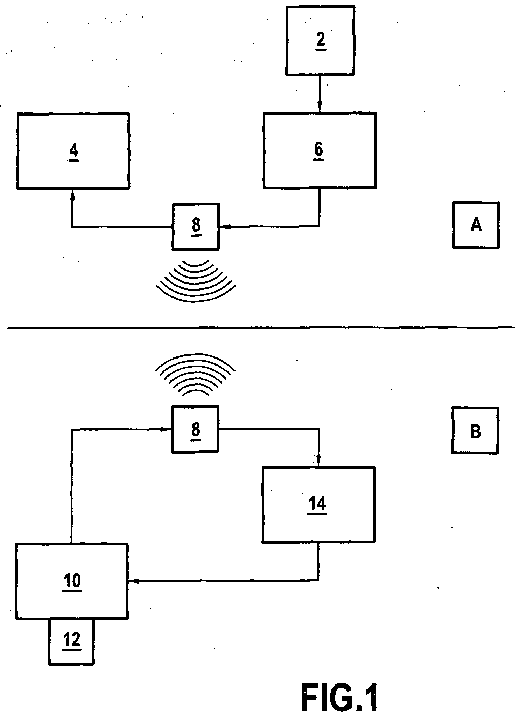

Un dispositif de télé-échographie est représenté sur la figure 1. II comporte d'une part du matériel sur un site A où un expert médical peut diriger à distance l'échographie et d'autre part du matériel embarqué dans un site B où se trouve le patient à examiner.

Le site d'expertise A comporte une sonde fictive 2 que l'expert médical peut manier à sa guise en fonction du retour d'images échographiques du patient qui lui est fait sur le moniteur 4. Les déplacements de la sonde fictive 2 sont mesurés par des capteurs intégrés dans cette sonde et les valeurs mesurées sont transmises à un poste de traitement 6, qui code ces valeurs en une trame informatique de manière à pouvoir les communiquer via des émetteurs récepteurs 8 depuis le site d'expertise A vers le site opératoire B.

Le site opératoire B comporte un système robotisé 10 adapté à réaliser l'échographie sur le patient en fonction des informations de commande qui découlent du déplacement de la sonde fictive sur le site d'expertise A.

Une sonde réelle 12, disposée en bout du système robotisé, permet d'obtenir des images échographiques qui sont envoyées via les émetteurs récepteurs, cette fois depuis le site d'opération B vers le site d'expertise A, vers le moniteur 4 pour que l'expert médical puisse visionner ces images et adapter le mouvement sur la sonde fictive pour collecter d'autres images.

Un module de commande 14 est adapté à piloter le système robotisé pour reproduire les mouvements de la sonde fictive 2. A cet effet, le module de commande reçoit, via les émetteurs récepteurs 8, les valeurs codées représentatives du déplacement de la sonde fictive.

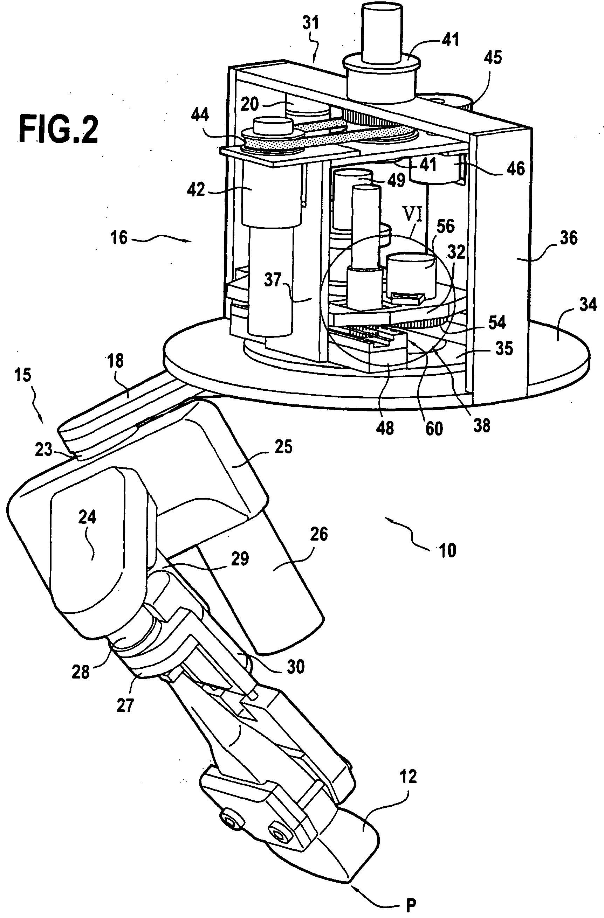

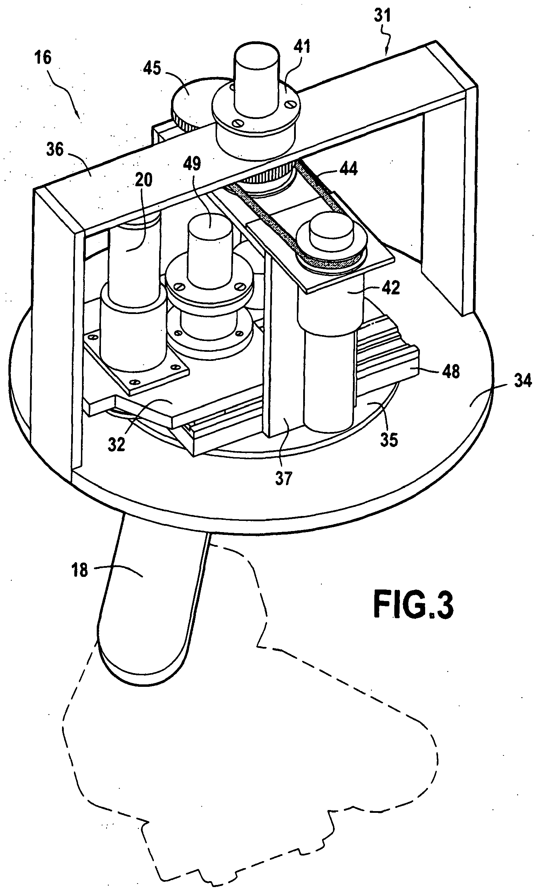

Tel qu'illustré sur la figure 2, le système robotisé 10 comporte un ensemble de bras articulés 15 portant à une extrémité la sonde réelle d'échographie 12, cet ensemble étant monté sur un support 16. L'ensemble 15 et le support 16 sont adaptés à être pilotés en fonction de signaux de commande en provenance du poste de commande 14, visible sur la figure 1. On observe que pour des raisons pratique de lecture des figures 2 à 6, les câbles permettant la transmission des signaux de commande, de même que les câbles d'alimentation, n'ont pas été représentés.

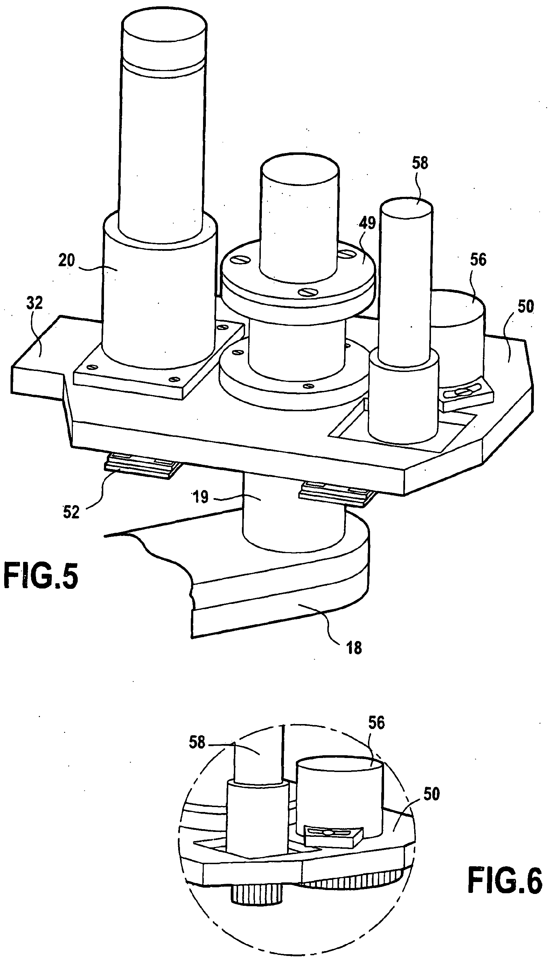

L'ensemble de bras articulés 15 comporte un bras supérieur 18 solidaire en rotation d'un premier arbre 19 du système monté pivotant sur le support (et particulièrement visible sur la figure 5). Ce bras supérieur comporte une première partie qui s'étend sous le support radialement au premier arbre et une deuxième partie qui prolonge dans la direction radiale du bras cette première partie à l'opposé du premier arbre, en formant avec celle-ci un angle déterminé.

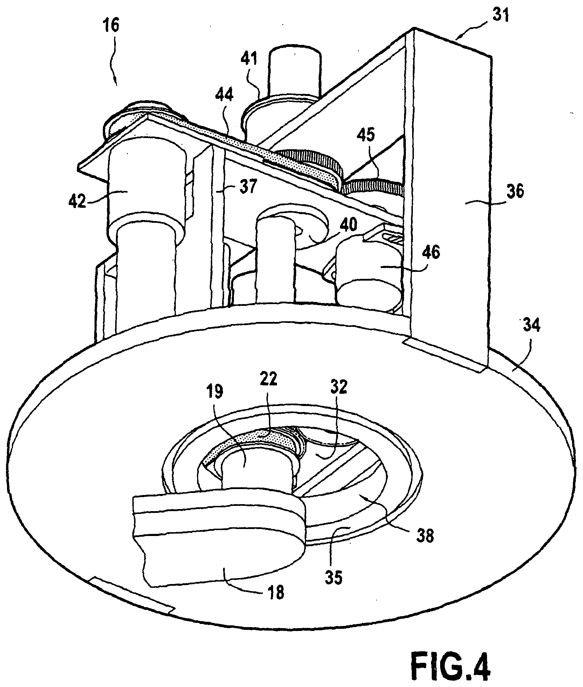

Un premier actionneur 20 est relié à ce premier arbre par une courroie 22 (notamment visible sur la figure 4) pour l'entraîner en rotation et pour ainsi commander la rotation du bras supérieur, dont l'extrémité libre de la deuxième partie comporte un deuxième arbre de rotation 23 monté pivotant et solidaire d'un bras intermédiaire 24.

Ce bras intermédiaire est associé à un deuxième actionneur 26 et une courroie (non visible ici car cachée par un carter 25) qui pilotant la rotation du bras intermédiaire par rapport au bras supérieur.

Un bras porte-outil 27 est monté solidaire en rotation d'un troisième arbre de rotation porté par le bras intermédiaire et il est associé à un troisième actionneur 29.

Le bras intermédiaire et le bras porte-outil sont respectivement associés à un capteur de position angulaire absolue, afin d'assurer la bonne position des bras les uns par rapport aux autres pour que l'orientation de la sonde en bout de l'ensemble des bras articulés soit bonne.

La sonde réelle 12 est montée à rotation sur ce bras porte-outil avec un degré de liberté en translation piloté par un quatrième actionneur 30, afin de pouvoir d'une part plaquer la sonde contre le corps du patient et s'assurer que le point P est toujours en contact de celui-ci quelle que soit l'inclinaison de la sonde pour l'obtention de bonnes images et d'autre part dégager légèrement la sonde du corps du patient si cela est nécessaire pour réaliser l'inclinaison de la sonde réelle.

Les trois arbres de rotation décrits ci-dessus tournent autour d'axes concourants en un point fixe P correspondant à l'extrémité de la sonde réelle 12, adaptée à être au contact de la zone de travail.

Le bras intermédiaire présente une forme sensiblement équivalente à celle du bras supérieur, à savoir une première partie perpendiculaire au deuxième arbre de rotation 23 et une deuxième partie inclinée. L'empattement et l'angle formé entre les deux parties du bras intermédiaire sont déterminés pour que la sonde puisse prendre une position, ici verticale, dans le sens de l'axe formé par le premier arbre, c'est à dire avec un premier arbre et un troisième arbre de rotation qui se retrouvent alignés. Comme cela sera décrit ci-après, le déplacement en rotation de la sonde autour de ce point fixe est obtenu par la commande en rotation de chacun des bras les uns par rapport aux autres et par rapport au support. En cours d'examen échographique, le point fixe P de la sonde réelle correspond au point de contact de cette sonde avec le corps du patient formant ici une zone de travail sur laquelle est adaptée à se déplacer la sonde.

Le support 16 est visible sur l'ensemble des figures 2 à 4, et des détails de ce support sont visibles sur les figures 5 et 6. Il comporte un portique 31 à l'intérieur duquel est montée coulissante une platine 32 qui porte entre autre le premier arbre 19 de l'ensemble de bras articulés 15.

Le portique 31 comporte d'une part une base fixe 34, de forme annulaire et surmontée d'une première arche 36, et d'autre part un plateau annulaire concentrique 35 adapté à tourner à l'intérieur de la base dans le plan défini par celle-ci et surmonté d'une deuxième arche 37. Le plateau 35 présente un diamètre extérieur sensiblement égal au diamètre interne de l'anneau formant la base fixe, et la deuxième arche 37 est de moindre hauteur que la première arche 36, de sorte que le plateau 35 peut tourner à l'intérieur de la base avec la deuxième arche qui passe sous la première arche. Le plateau 35 est percé en son centre d'un trou 38 de diamètre déterminé, et il sera compris ci-après que la valeur de ce diamètre permet un plus ou moins long débattement en translation du système robotisé.

Un arbre d'entraînement 40 (notamment visible sur la figure 4) est solidaire du plateau 35 via la deuxième arche 37 et il est couplé à un collecteur tournant 41 rapporté sur la première arche 36 solidaire de la base 34 et dont les avantages d'utilisation seront décrits ci-après. Cette base reste fixe et le plateau tourne à l'intérieur de cette base, suite à la mise en rotation de l'arbre d'entraînement 40 par l'action d'un cinquième actionneur 42 fixé sur la deuxième arche 37 et d'une courroie 44 qui relie une poulie solidaire du cinquième actionneur et une poulie disposée sur le pourtour du collecteur tournant 41 solidaire de la première arche.

Ce collecteur tournant 41 comporte une partie fixe solidaire de la première arche et dont le pourtour comporte, outre la poulie pour l'entraînement par courroie, une couronne dentée adaptée à s'engrener avec un pignon 45 monté sur la deuxième arche et associé à un capteur de position angulaire 46 qui détermine la position absolue du plateau. Le collecteur tournant comporte en outre une partie mobile (non visible sur les figures) en rotation à l'intérieur de la partie fixe et qui est solidaire en rotation de l'arbre d'entraînement 40 et de la deuxième arche 37.

Comme cela sera décrit ci-après, le plateau 35 est adapté à tourner selon un angle déterminé, et la platine 32 est montée de manière à suivre cette rotation, entraînant de la sorte en rotation l'ensemble de bras articulés. Dans chacune des positions angulaires du plateau, la platine peut être pilotée en translation par rapport à ce plateau, entraînant de la sorte en translation l'ensemble de bras articulés.

Deux rails 48 parallèles sont fixés à cet effet sur le plateau 35, de part et d'autre du trou 38, en étant adaptés à recevoir la platine.

Cette platine 32 présente la forme d'une plaque 50 sensiblement plane, qui porte sensiblement en son centre un collecteur tournant 49 associé au premier arbre 19. La platine 32 est adaptée à coulisser dans les rails 48 par l'intermédiaire de coulisseaux 52 rapportés sous la plaque 50. On dénombre ici deux coulisseaux par rail.

Comme décrit précédemment, le premier actionneur 20 est adapté par l'intermédiaire de la courroie associée 22 à entraîner en rotation le premier arbre 19 par rapport à la platine. Celle-ci comporte en outre un pignon 54 qui est montée sous la platine et qui est adapté à coopérer avec une couronne dentée s'étendant autour du premier arbre 19 pour permettre le contrôle de la position angulaire du premier axe. Ce pignon 54 est associé à un capteur de position angulaire 56 qui détermine la position angulaire absolue du premier arbre 19 de l'ensemble de bras articulés.

La platine comporte en outre un sixième actionneur 58 qui commande la rotation d'un pignon d'engrenage 59 (visible sur la figure 6 et représenté en traits pointillés sur la figure 2) et qui est disposé sous la plaque pour coopérer avec une denture droite formant crémaillère sur un bord extérieur 60 le long d'un rail 48. De la sorte, comme cela sera décrit ci-après, la platine peut coulisser par rapport au plateau, dans l'axe des rails.

On va maintenant décrire le cas spécifique d'un déplacement en translation de la sonde réelle et du point de contact à son extrémité, suite à un mouvement de translation commandé à distance par l'expert médical. A cet effet, le module de commande élabore d'une part un signal de déplacement angulaire, qu'il transmet au cinquième actionneur 42, et d'autre part un signal de déplacement radial, qu'il transmet au sixième actionneur 58 associé à la platine du portique.

Le cinquième actionneur 42 est commandé pour entraîner en rotation, via la courroie 44, l'arbre d'entraînement solidaire du plateau. Le plateau pivote ainsi par rapport à la base du portique, ce qui génère une rotation de l'ensemble de bras articulés et une orientation différente de la sonde.

On observe que sur le site expert A, la sonde virtuelle a été déplacée en translation par l'expert médical en conservant la même inclinaison et sans aucune rotation. Pour reproduire ce résultat final d'une pure translation, sans composante rotative, le module de commande élabore de fait un signal complémentaire de commande du premier actionneur 20 pour la mise en rotation du bras supérieur du robot, afin de compenser la rotation de la sonde induite lors du déplacement angulaire de la platine et d'orienter ainsi correctement la tête de sonde par rapport à l'orientation de la sonde fictive manoeuvrée par l'expert médical.

On constate sur les figures 2 à 4 que le bras supérieur est agencé de sorte que son axe de rotation soit parallèle à l'axe de l'arbre d'entraînement 40. La rotation de la sonde induite par le déplacement angulaire de la platine est une rotation autour de l'axe de rotation du plateau supportant la platine c'est à dire autour de l'axe défini par l'arbre d'entraînement 40. Une rotation induite d'une valeur d'angle α sera alors compensée par une rotation du bras supérieur d'une valeur d'angle inverse -α autour de son axe de rotation défini par le premier arbre 19 et parallèle à l'arbre d'entraînement 40.

A ce stade, la platine a été pivotée d'un angle α correspondant à la coordonnée angulaire du déplacement en translation souhaitée et il convient de déplacer en translation cette platine, porteuse de l'ensemble de bras articulés et de la sonde, de la coordonnée radiale déterminée par le module de commande.

A cet effet, le sixième actionneur 58 est piloté pour entraîner en rotation le pignon associé qui est en prise avec la crémaillère solidaire d'un des rails. L'engrenage pignon-crémaillère permet le déplacement de la platine le long des rails. Le trou 38 par lequel passe le premier arbre 19 du système permet le déplacement radial de ce premier arbre en suivant le déplacement de la platine. Le trou présente un diamètre déterminé correspondant au débattement en translation autorisé de la sonde. Il s'agit dès lors pendant la conception du robot de trouver un compromis entre le souhait d'avoir un débattement en translation suffisant du premier arbre dans le trou et donc de la sonde sur le corps du patient, et celui d'avoir un système robotisé compact.

II en résulte que le premier arbre 19, et donc l'ensemble de bras articulés, a été translaté dans le plan de la platine par un déplacement angulaire et un déplacements radial. Lors de cette translation, la tête de sonde est restée orientée correctement par rapport à l'orientation de la sonde fictive, grâce à la compensation commandée par le module 14.

On comprend de ce qui précède que pour la prise en charge d'une translation de la sonde le long du corps du patient, le système robotisé selon l'invention présente l'avantage d'utiliser des signaux de commande relatifs à des coordonnées polaires, notamment pour un souci de compacité du robot. La rotation induite de la sonde lors du déplacement angulaire du support spécifique au mouvement souhaité de translation de cette sonde est gérée et compensée par la conception du robot et au moins par l'agencement du bras supérieur et du premier arbre directement rapporté sur le support, qui permet de compenser cette rotation induite alors qu'elle n'existe pas lorsque l'on envisage d'utiliser des signaux de commande cartésiens comme cela est connu.

On va maintenant décrire l'utilisation du système robotisé selon l'invention dans des examens échographiques.

Sur le site opératoire, on positionne le robot de telle sorte que l'extrémité de la sonde réelle d'échographie 12 soit au contact de la zone de travail, ici le corps du patient, en regard de la zone à examiner.

Les images échographiques prises par la sonde sont envoyées via les émetteurs récepteurs vers le moniteur présent sur le site d'expertise et ces images sont analysées par l'expert médical.

Afin de parfaire son analyse, l'expert médical cherche à obtenir d'autres images soit en inclinant la sonde par rapport à son point de contact, soit en déplaçant ce point de contact. Pour ce faire, l'expert médical réalise des mouvements correspondants sur la sonde fictive pour que ceux-ci soient reproduits sur le site opératoire via le dispositif robotisé.

Les mouvements de rotation et de translation de la sonde fictive sont traduits et transmis au module de commande via le poste de traitement et les émetteurs récepteurs comme cela a été décrit précédemment par la création d'une trame informatique à base d'octets représentative de ces mouvements. Le module de commande détermine alors quel doit être les mouvements du support et de chacun des bras de l'ensemble de bras articulés pour reproduire fidèlement le déplacement de la sonde en bout du système robotisé, et il élabore des signaux de commande correspondants pour chacun des six actionneurs du système robotisé, c'est à dire pour chacun des quatre actionneurs associés à l'ensemble de bras articulés et pour chacun des deux actionneurs associés au support. On comprendra que les matrices de calcul intégrées dans le module de commande et adaptées à transformer le mouvement souhaité de la sonde en des signaux de commande des bras du système robotisé sont connues et qu'elles pourraient prendre des valeurs différentes pour s'adapter à différents types de robot et notamment à des agencements particuliers des bras.

Avantageusement, afin de pouvoir gérer en temps réel un possible déplacement de la sonde simultanément en translation et en rotation, le module de commande calcule en permanence des instructions pour l'ensemble des actionneurs du système robotisé.

Les mouvements opérés par l'expert médical sur la sonde fictive de pilotage sont ainsi reproduits à distance par la sonde réelle qui se déplacent sur le corps du patient et permet la prise de vue d'images échographiques.

En temps réel, le module de commande reçoit d'une part les instructions de déplacement de la sonde réelle, pour les transmettre en autant de signaux de commande que nécessite le cas d'espèce et il reçoit d'autre part des informations en provenance des actionneurs et des capteurs de position angulaire de manière à avoir en temps réel une donnée fiable quant au positionnement de chacun des bras et du support, afin de pouvoir calculer les signaux de commande adéquats par la suite pour piloter le robot en accord avec les instructions de l'expert médical.

Si l'expert médical souhaite uniquement une inclinaison de la sonde autour du point fixe matérialisé par le contact de cette sonde avec le patient en conservant la position de ce point fixe, le module de commande envoie un signal de commande à chacun des actionneurs disposés dans l'ensemble de bras articulés, c'est à dire au premier actionneur associé au bras supérieur, au deuxième actionneur associé au bras intermédiaire et au troisième actionneur associé au bras porte-outil ainsi qu'au quatrième actionneur pour la translation de la sonde le long de l'axe du bras porte-outil, contre le corps du patient. Dans le même temps, les signaux de commande correspondants aux cinquième et sixième actionneurs restent inchangés, aucune translation de l'ensemble des bras n'étant souhaitée.

Si l'expert médical souhaite uniquement une translation du point fixe en gardant la même inclinaison, le module de commande élabore simultanément un signal de déplacement angulaire, qu'il transmet au cinquième actionneur, un signal de déplacement radial, qu'il transmet au sixième actionneur, ainsi que, comme cela a été décrit précédemment, un signal complémentaire de commande pour le premier actionneur, inverse au signal de déplacement angulaire transmis au cinquième actionneur, pour que le premier actionneur compense la rotation de la sonde lors du déplacement angulaire de la platine. Dans le même temps, les signaux de commande correspondants aux deuxième, troisième et quatrième actionneurs restent inchangés.

On constate que dans les deux cas, le premier actionneur est toujours piloté. Si l'expert médical souhaite déplacer le point de contact de la sonde et dans le même temps modifier l'inclinaison de la sonde autour de ce point fixe, de nouveaux signaux de commande sont envoyés simultanément vers les six actionneurs et le signal de commande envoyé au premier actionneur correspond à l'addition des deux signaux de commande prévus respectivement pour l'inclinaison lors de la rotation de la sonde et pour la compensation angulaire lors de la translation de la sonde. On comprendra qu'on entend ici par l'addition d'un signal de commande et d'un signal complémentaire de commande une addition ou une soustraction des valeurs absolues de ces signaux, pour tenir compte des valeurs d'angle éventuellement négatives par rapport à une position donnée du bras supérieur.

A la lecture de ce qui précède, on constate aisément que l'invention atteins bien les buts qu'elle s'est fixés, et qu'il est inutile de rappeler entièrement. Ainsi, on propose un système robotisé qui permet à un médecin spécialiste de réaliser une échographie et d'établir un diagnostic fiable sur un patient distant. L'enjeu médical est de prolonger les mouvements de la main du médecin à un robot dédié et dans le cas présent de permettre de suivre les mouvements d'ajustement en translation que souhaite faire le médecin pour une plus grande visibilité. Ce suivi efficace du mouvement du médecin est réalisé avec un système compact permettant son utilisation dans n'importe quelles conditions et n'importe quel lieu. L'utilisation d'une commande en polaire et la structure associée du support du robot permettent de réduire l'empattement du système en gardant les mêmes fonctionnalités et notamment un débattement en translation donné.

La position de la sonde est dès lors reproduite en temps réel, avec une position du point de contact qui respecte le déplacement en translation de la main du médecin, une inclinaison de la sonde par rapport à la zone de travail et ce point de contact qui est respectée également, et une orientation de la sonde qui n'est pas impactée par la prise en compte de la translation, du fait de la compensation angulaire qui est faite en temps réel sur la commande en rotation du bras supérieur, autour d'un axe parallèle à l'axe de rotation d'un élément tournant du support.

La présence des collecteurs tournants permet de dissocier les câbles électriques rapportés sur la partie fixe et ceux rapportés sur la partie tournante. Il est dès lors possible de tourner indifféremment dans un sens de rotation ou dans l'autre sans se soucier d'un nombre de tours maximal dans un même sens à respecter pour éviter que les câbles ne se brisent et que le contact électrique soit rompu. Comme cela a été décrit précédemment, pour des raisons pratique de lecture des figures, les câbles n'ont pas été représentés, mais on comprendra qu'ils sont associés aux parties fixe et tournante des collecteurs tournants de façon connue par ailleurs. Ici, l'utilisation de collecteurs tournants est avantageuse en ce que le module de commande n'a pas de contraintes mathématiques supplémentaires pour déterminer s'il faut tourner telle ou telle partie du collecteur dans tel ou tel sens. Le collecteur tournant 49 associé au premier arbre 19 est particulièrement intéressant en ce qu'il permet de rendre indépendant les deux niveaux que sont le support et l'ensemble des bras articulées Le module de commande peut faire tourner les bras dans un sens sans se soucier du sens dans lequel va tourner le plateau du support.

Dans des variantes non représentées, on pourra prévoir, sans que cette liste soit exhaustive, que :
- le robot porteur de la sonde réelle est réalisé autrement dès lors qu'il est associé à un support permettant son déplacement en translation selon des coordonnées polaires ; il convient ici que le robot présente un bras supérieur monté à rotation autour d'un axe parallèle à l'axe de rotation correspondant à la commande en déplacement angulaire du support dans le cadre de sa translation en coordonnées polaires;
- à titre d'exemple, la sonde réelle 12 peut être montée sur le bras porte-outil sans être piloté par un actionneur dédié comme cela a été décrit précédemment avec le quatrième actionneur, mais en faisant plutôt l'objet d'un montage passif avec un système de ressort de rappel tendant à ramener l'extrémité de la sonde en contact avec la zone de travail. On comprendra que dès lors, sans sortir du contexte de l'invention, le module de commande élabore des signaux adaptés à chaque situation vue précédemment pour cinq actionneurs au lieu de six ;
- les capteurs de position angulaire associés à chacun des bras du robot peuvent être des capteurs relatifs calés à l'initialisation. On comprendra que dans le mode de réalisation décrit, l'intérêt d'utiliser des capteurs de position absolue est qu'ils permettent d'éviter une étape de calibrage ;
- le site d'expertise ne comporte pas de poste de commande et que le module de commande disposé sur le site opératoire reçoit directement, via les émetteurs récepteurs, les valeurs mesurées par les capteurs intégrés dans la sonde fictive ;
- le site opératoire comporte également un moniteur échographique et ce moniteur envoie en sortie des images vidéos via les émetteurs récepteurs vers le moniteur sur lequel l'expert médical s'appuie pour son examen;
- l'expert médical peut donner ses instructions à distance par différents types d'outils fictifs. S'il est particulièrement intéressant pour l'expert de pouvoir manier une sonde unique capable de transmettre des informations quant à la commande en translation et en rotation souhaitée, il pourra être prévu, pour une conception simplifiée, que l'expert médical manie une sonde fictive pour déterminer l'inclinaison à donner et qu'il manie simultanément un manche de type "joystick" pour piloter la translation de la sonde.

Ainsi, l'invention n'est pas limitée au seul dispositif conforme au mode de réalisation explicitement décrit en regard des figures 1 à 6, et il convient également de noter que l'invention n'est pas limitée à l'application préférée relative à des opérations de télé-échographie. Elle peut également être utilisée pour des manipulations d'outils dans des conditions d'usinage qui nécessitent un centre de commandement délocalisé, par exemple dans des environnements hostiles.

## Revendications

1. Système robotisé (10) du type comportant un support (16) sur lequel est rapporté un ensemble de bras articulés (15) à l'extrémité duquel est disposé un outil (12) adapté à être en contact avec une surface de travail déterminée, ledit système étant adapté à déplacer l'outil en rotation autour du point de contact (P) de l'outil avec ladite surface de travail en conformité avec des instructions de déplacement en provenance d'un modulé de commande (14) adapté à calculer ces instructions en fonction du déplacement à distance d'un outil de pilotage virtuel dudit système robotisé, le système étant adapté en outre à déplacer en translation le point de contact de l'outil, **caractérisé en ce que** cette translation est commandée par le module de commande en cordonnées polaires, de sorte que ladite translation du point de contact de l'outil est réalisée par un déplacement angulaire d'éléments constitutifs dudit support suivi d'un déplacement radial d'éléments constitutifs de ce même support.

2. Système robotisé selon la revendication 1, **caractérisé en ce que** les éléments constitutifs du support comportent un plateau (35) adapté à tourner sur lui-même pour la réalisation dudit déplacement angulaire ainsi qu'une platine (32) qui porte l'extrémité dudit ensemble de bras articulés (15) et qui est montée en translation dans des rails (48) solidaires du plateau pour la réalisation dudit déplacement radial.

3. Système robotisé selon la revendication 2, **caractérisé en ce que** la platine (32) porte un actionneur (58) entraînant en rotation un pignon (59) adapté à coopérer avec une crémaillère formée sur un des rails (48) solidaires du plateau, ledit actionneur étant piloté par un signal de commande en provenance du module de commande (14) pour la réalisation dudit déplacement radial.

4. Système robotisé selon l'une des revendications 2 ou 3, **caractérisé en ce que** le plateau (35) comporte un trou (38) qui est disposé entre les rails (48) et par lequel passe un premier arbre de rotation (19) monté pivotant sur le support (16) et solidaire de l'ensemble de bras articulés (15), le trou présentant un diamètre adapté pour permettre ledit déplacement radial.

5. Système robotisé selon l'une des revendications précédentes, **caractérisé en ce que** les éléments constitutifs du support comportent en outre une base (34) fixe d'un portique (31) à l'intérieur duquel tourne le plateau (35), ladite base fixe portant un arbre d'entraînemenf (40) qui est monté à rotation sur ladite base, qui est solidaire en rotation du plateau et qui est associé à un actionneur (42), ledit actionneur étant piloté par un signal de commande en provenance du module de commande (14) pour la réalisation dudit déplacement angulaire.

6. Système robotisé selon la revendication 1, **caractérisé en ce que** le module de commande (14) génère un signal de commande en rotation d'au moins un bras dudit ensemble de bras articulés (15) porteur de l'outil, ledit signal de commande étant adapté à compenser la rotation de l'outil induite par son déplacement angulaire lors de sa translation polaire.

7. Système robotisé selon la revendication 6 dans lequel l'ensemble de bras articulés (15) comporte au moins un bras supérieur (18) qui est solidaire en rotation d'un premier arbre (19) du système monté pivotant sur le support (16), le module de commande (14) générant une rotation dudit bras supérieur (18) pour compenser ladite rotation induite de l'outil, **caractérisé en ce que** l'axe de rotation du bras supérieur est parallèle à l'axe du déplacement angulaire du support.

8. Système robotisé selon la revendication 7, **caractérisé en ce que** le premier arbre (19) est monté sur le support couplé à un collecteur tournant (49) solidaire dudit support.

9. Système robotisé selon la revendication 7 ou 8, dans lequel l'ensemble de bras articulés (15) comporte en outre un bras intermédiaire (24) solidaire d'un deuxième arbre de rotation (23) monté pivotant à l'extrémité libre dudit bras supérieur (18), ledit bras intermédiaire portant un troisième arbre de rotation (28) duquel est solidaire en rotation un bras porte-outil (27) sur lequel est monté l'outil, et dans lequel trois axes de rotation sont définis respectivement par le premier arbre (19), le deuxième arbre (23) et le troisième arbre (28) de rotation, lesdits trois axes étant concourants en l'extrémité de l'outil, **caractérisé en ce que** le module de commande (14) est adapté à fournir d'une part des signaux de commande à destination d'actionneurs (20, 26, 29) associés respectivement à chacun des bras articulés pour piloter la rotation des bras articulés autour de chacun des trois axes afin de commander le déplacement en rotation de l'outil et à fournir d'autre part à l'actionneur associé au bras supérieur un signal complémentaire inverse au signal correspondant au déplacement angulaire, pour compenser la rotation de l'outil induite par le déplacement angulaire dudit support pour le déplacement en translation de l'outil.

10. Système robotisé selon les revendications 3, 5 et 9 prises en combinaison, **caractérisé en ce que** chaque actionneur (20, 26, 29, 42, 58) est associé à un capteur de position angulaire adapté à déterminer la position angulaire absolue du bras, de l'arbre ou du pignon piloté par ledit actionneur.

11. Dispositif de télé-échographie dans lequel on réalise à distance le déplacement d'une sonde d'échographie (12), le dispositif de télé-échographie comportant d'une part du matériel sur un site d'expertise (A) où un expert médical peut diriger à distance l'échographie et d'autre part du matériel embarqué dans un site opératoire (B) où se trouve le patient à examiner, le site opératoire comportant un système robotisé (10) selon l'une des revendications 1 à 10 adapté à réaliser l'échographie sur le patient en fonction des informations de commande qui découlent du déplacement d'une sonde virtuelle (2) sur le site d'expertise.

## Patentansprüche

1. Robotersystem (10), das Folgendes aufweist: einen Träger (16), auf dem eine Gruppe von Gelenkarmen (15) angebracht ist, an deren Ende ein Werkzeug (12) angeordnet ist, das zum Kontakt mit einer bestimmten Arbeitsfläche ausgelegt ist, wobei das System das Werkzeug veranlassen kann, eine Drehbewegung um den Punkt des Kontakts (P) mit der Arbeitsfläche entsprechend Bewegungsanweisungen aus einem Steuermodul (14) auszuführen, das in der Lage ist, diese Anweisungen entsprechend der Bewegung ferngesteuert durch ein virtuelles Steuerwerkzeug des Robotersystems zu berechnen, wobei das System ferner in der Lage ist, den Kontaktpunkt des Werkzeugs translatorisch zu bewegen, **dadurch gekennzeichnet, dass** diese Translation durch das Steuermodul in Polarkoordinaten gesteuert wird, derart dass die Translation des Kontaktpunkts des Werkzeugs durch eine Winkelbewegung von Bestandselementen des Trägers und eine anschließende Radialbewegung von Bestandselementen dieses gleichen Trägers realisiert wird.

2. Robotersystem nach Anspruch 1, **dadurch gekennzeichnet, dass** die Bestandselemente des Trägers ein Tablett (35), das so gestaltet ist, dass es sich zum Ausführen der Winkelbewegung um sich selbst drehen kann, sowie eine Auflageplatte (32) aufweisen, die das äußerste Ende der Gruppe von Gelenkarmen (15) trägt und die zum Ausführen der Radialbewegung translatorisch auf mit dem Tablett fest verbundenen Schienen (48) montiert ist.

3. Robotersystem nach Anspruch 2, **dadurch gekennzeichnet, dass** die Auflageplatte (32) ein Stellglied (58) trägt, das ein Zahnrad (59) in Drehung versetzt, das dazu ausgelegt ist, mit einer Zahnleiste zusammenzuwirken, die auf einer der mit dem Tablett fest verbundenen Schienen (48) ausgebildet ist, wobei das Stellglied von einem Steuersignal aus dem Steuermodul (14) zum Ausführen der Radialbewegung gesteuert wird.

4. Robotersystem nach einem der Ansprüche 2 oder 3, **dadurch gekennzeichnet, dass** das Tablett (35) ein Loch (38) aufweist, das sich zwischen den Schienen (48) befindet und durch das eine erste Antriebswelle (19) hindurchragt, die schwenkbar auf dem Träger (16) montiert und mit der Gruppe von Gelenkarmen (15) fest verbunden ist, wobei das Loch einen Durchmesser aufweist, der eine Radialbewegung ermöglicht.

5. Robotersystem nach einem der vorangehenden Ansprüche, **dadurch gekennzeichnet, dass** die Bestandselemente des Trägers des Weiteren einen festen Sockel (34) eines Rahmens (31) aufweisen, in dessen Inneren sich das Tablett (35) dreht, wobei der feste Sockel eine Antriebswelle (40) trägt, die auf dem Sockel drehbar befestigt ist, der mit dem Tablett drehfest verbunden ist, und die einem Stellglied (42) zugehörig ist, wobei das Stellglied von einem Steuersignal aus dem Steuermodul (14) zum Ausführen der Winkelbewegung gesteuert wird.

6. Robotersystem nach Anspruch 1, **dadurch gekennzeichnet, dass** das Steuermodul (14) ein Rotationsbefehlssignal von mindestens einem Arm der Gesamtheit von Gelenkarmen (15) erzeugt, der das Werkzeug trägt, wobei das Steuersignal dazu dient, die Drehung des Werkzeugs zu kompensieren, die durch seine Winkelbewegung während seiner Translation in Polarkoordinaten hervorgerufen wird.

7. Robotersystem nach Anspruch 6, wobei die Gruppe von Gelenkarmen (15) mindestens einen oberen Arm (18) aufweist, der mit einer ersten Welle (19) des schwenkbar auf dem Träger (16) montierten Systems drehfest verbunden ist, wobei das Steuermodul (14) eine Drehung des oberen Arms (18) erzeugt, um die durch das Werkzeug hervorgerufene Drehung zu kompensieren, **dadurch gekennzeichnet, dass** die Rotationsachse des oberen Arm parallel zur Achse der Winkelbewegung des Trägers verläuft.

8. Robotersystem nach Anspruch 7, **dadurch gekennzeichnet, dass** die erste Welle (19) auf dem Träger montiert ist, der mit einem Schleifring (49) gekoppelt ist, der mit dem Träger fest verbunden ist.

9. Robotersystem nach Anspruch 7 oder 8, wobei die Gruppe von Gelenkarmen (15) des Weiteren einen Zwischenarm (24) aufweist, der mit einer zweiten Welle (23) fest verbunden ist, die schwenkbar am freien Ende des oberen Arms (18) befestigt ist, wobei der Zwischenarm eine dritte Welle (28) trägt, mit der ein Werkzeugtragearm (27), an dem das Werkzeug montiert ist, drehfest verbunden ist, und wobei drei Rotationsachsen jeweils durch die erste Welle (19), die zweite Welle (23) und die dritte Welle (28) definiert sind, wobei die drei Achsen am äußeren Ende des Werkzeugs konvergieren, **dadurch gekennzeichnet, dass** das Steuermodul (14) dazu dient, einerseits für die Stellglieder (20, 26, 29), die den jeweiligen Gelenkarmen zugehörig sind, bestimmte Steuersignale bereitzustellen, um die Drehung der Gelenkarme um jede der drei Achsen zu steuern, um die Drehbewegung des Werkzeugs zu steuern, und um andererseits für das Stellglied, das dem oberen Arm zugehörig ist, ein Komplementärsignal bereitzustellen, das in Bezug auf das der Winkelbewegung entsprechende Signal invers ist, um für die Tanslationsbewegung des Werkzeugs die durch die Winkelbewegung des Trägers hervorgerufene Drehung des Werkzeugs zu kompensieren.

10. Robotersystem nach den Ansprüchen 3, 5 und 9 in Kombination, **dadurch gekennzeichnet, dass** jedes Stellglied (20, 26, 29, 42, 58) einem Winkelpositionssensor zugehörig ist, der dazu ausgelegt ist, die absolute Winkelposition des Arms, der Welle oder des durch das Stellglied gesteuerten Zahnrads zu bestimmen.

11. Telesonographie-Vorrichtung, wobei die Bewegung einer Ultraschallsonde (12) ferngesteuert wird, wobei die ferngesteuerte Telesonographie-Vorrichtung einerseits Gerätschaft in einem Kompetenzzentrum (A) aufweist, wo ein medizinischer Fachmann die Ultraschallsonde fernsteuern kann, und andererseits Gerätschaft aufweist, die an einem Ausführungsort (B) aufgestellt wurde, wo sich der zu untersuchende Patient befindet, wobei der Ausführungsort das Robotersystem (10) nach einem der Ansprüche 1 bis 10 aufweist, das in der Lage ist, Ultraschalluntersuchungen an dem Patienten entsprechend den Befehlsinformationen auszuführen, die von der Bewegung einer virtuellen Sonde (2) in dem Kompetenzzentrum herrühren.

## Claims

1. Robotised system (10) of the type comprising a support (16) to which is attached a set of articulated arms (15) at the end of which is arranged a tool (12) adapted to be in contact with a determined work surface, said system being adapted to move the tool in rotation about the point of contact (P) of the tool with said work surface in accordance with movement instructions originating from a control module (14) adapted to calculate these instructions as a function of the remote movement of a virtual driving tool of said robotised system, the system further being adapted to move the point of contact of the tool in translation, **characterised in that** this translation is controlled by the control module in polar coordinates, so that said translation of the point of contact of the tool is produced by an angular movement of constituent elements of said support followed by a radial movement of constituent elements of this same support.

2. Robotised system according to claim 1, **characterised in that** the constituent elements of the support comprise a plate (35) adapted to rotate on itself to produce said angular movement as well as a deck (32) which carries the end of said set of articulated arms (15) and which is mounted in translation in rails (48) securely attached to the plate to produce said radial movement.

3. Robotised system according to claim 2, **characterised in that** the deck (32) carries an actuator (58) driving in rotation a pinion (59) adapted to cooperate with a rack formed on one of the rails (48) securely attached to the plate, said actuator being driven by a control signal originating from the control module (14) to produce said radial movement.

4. Robotised system according to one of claims 2 or 3, **characterised in that** the plate (35) comprises a hole (38) which is arranged between the rails (48) and through which passes a first rotation shaft (19) mounted to pivot on the support (16) and securely attached to the set of articulated arms (15), the hole having a diameter adapted to allow said radial movement.

5. Robotised system according to one of the preceding claims, **characterised in that** the constituent elements of the support further comprise a fixed based (34) of a portal structure (31) inside which the plate (35) rotates, said fixed base carrying a driving shaft (40) which is mounted to rotate on said base, which is securely attached in rotation to the plate and which is associated with an actuator (42), said actuator being driven by a control signal originating from the control module (14) to produce said angular movement.

6. Robotised system according to claim 1, **characterised in that** the control module (14) generates a rotation control signal for at least one arm of said set of articulated arms (15) carrying the tool, said control signal being adapted to compensate for the rotation of the tool induced by its angular movement during its polar translation.

7. Robotised system according to claim 6, in which the set of articulated arms (15) comprises at least one top arm (18) which is securely attached in rotation to a first shaft (19) of the system mounted to pivot on the support (16), the control module (14) generating a rotation of said top arm (18) to compensate for said induced rotation of the tool, **characterised in that** the axis of rotation of the top arm is parallel to the axis of the angular movement of the support.

8. Robotised system according to claim 7, **characterised in that** the first shaft (19) is mounted on the support coupled to a rotating slip ring (49) securely attached to said support.

9. Robotised system according to claim 7 or 8, in which the set of articulated arms (15) further comprises an intermediate arm (24) securely attached to a second rotation shaft (23) mounted to pivot at the free end of said top arm (18), said intermediate arm carrying a third rotation shaft (28) to which there is securely attached in rotation a tool-holder arm (27) on which the tool is mounted, and in which three axes of rotation are defined respectively by the first rotation shaft (19), the second rotation shaft (23) and the rotation third shaft (28), said three axes intersecting each other at the tool end, **characterised in that** the control module (14) is adapted to supply, on the one hand, control signals to actuators (20, 26, 29) associated respectively with each of the articulated arms to drive the rotation of the articulated arms about each of the three axes in order to control the rotational movement of the tool, and, on the other hand, to supply to the actuator associated with the top arm, a complementary signal, the reverse of the signal corresponding to the angular movement, to compensate for the rotation of the tool induced by the angular movement of said support for the translational movement of the tool.

10. Robotised system according to claims 3, 5 and 9 taken in combination, **characterised in that** each actuator (20, 26, 29, 42, 58) is associated with an angular position sensor adapted to determine the absolute angular position of the arm, the shaft or the pinion driven by said actuator.

11. Remote echography device in which the movement of an echography probe (12) is remotely produced, the remote echography device comprising, on the one hand, equipment on an appraisal site (A) where a medical expert can remotely direct the echography and, on the other hand, equipment installed in an operating site (B) where the patient to be examined is located, the operating site comprising a robotised system (10) according to one of claims 1 to 10, adapted to perform the echography on the patient according to the control information derived from the movement of a virtual probe (2) in the appraisal site.
